# EUROPEAN PATENT APPLICATION

(11) **EP 4 491 142 A1**
(43) Date of publication of application: **15.01.2025**
(21) Application number: 23766615.1
(22) Date of filing: 27.02.2023
(51) Int. Cl.: A61B 34/10, A61B 34/37, G09B 9/00

(54) **SIMULATION SYSTEM, INFORMATION PROCESSING DEVICE, AND INFORMATION PROCESSING METHOD**

(30) Priority: 08.03.2022 JP 2022035336
(71) Applicant: Sony Group Corporation, Tokyo 108-0075 (JP)
(72) Inventor: KURODA, Yohei, Tokyo 108-0075 (JP); HEREDIA, Saul, Tokyo 108-0075 (JP)
(74) Representative: D Young & Co LLP
(86) International application number: PCT/JP2023/006943
(87) International publication number: WO 2023/171437

(57) **Abstract**

A simulation system (100) includes a first information processing apparatus (1) and a second information processing apparatus (2) that provide simulation of surgery in cooperation with each other. The first information processing apparatus (1) and the second information processing apparatus (2) transmit and receive simulation data to and from each other via an all-optical communication network (N).

## Description

### Field

The present disclosure relates to a simulation system, an information processing apparatus, and an information processing method.

### Background

In the field of surgery, it is known that simulation of surgery is performed (see, for example, Patent Literature 1).

### Citation List

### Patent Literature

Patent Literature 1: JP 2020-62494 A

### Summary

### Technical Problem

It is conceivable to use a high computing capability of cloud computing in order to improve simulation performance. In that case, it is necessary to deal with a delay in communication. In particular, in the case of simulation with a high real-time property involving sense-of-force presentation, the problem can become apparent.

One aspect of the present disclosure improves simulation performance.

### Solution to Problem

A simulation system according to one aspect of the present disclosure includes a first information processing apparatus, and a second information processing apparatus that provide simulation of surgery in cooperation with each other, wherein the first information processing apparatus and the second information processing apparatus transmit and receive simulation data to and from each other via an all-optical communication network.

An information processing apparatus according to one aspect of the present disclosure provides simulation of surgery in cooperation with another information processing apparatus, the information processing apparatus inluces: a processing unit that calculates a simulation model including a soft tissue; and an optical transmission control unit that controls transmission and reception of simulation data to and from the other information processing apparatus via an all-optical communication network.

An information processing method according to one aspect of the present disclosure is for providing simulation of surgery by a first information processing apparatus and a second information processing apparatus cooperating with each other, the information processing method includes, by the first information processing apparatus and the second information processing apparatus, transmitting and receiving simulation data to and from each other via an all-optical communication network.

### Brief Description of Drawings

FIG. 1 is a diagram schematically illustrating an overview of a simulation system according to an embodiment.
FIG. 2 is a diagram illustrating an example of functional blocks of the simulation system.
FIG. 3 is a block diagram illustrating an example of a schematic configuration of a haptic device.
FIG. 4 is a diagram illustrating an example of a video.
FIG. 5 is a diagram illustrating an example of a simulation loop.
FIG. 6 is a diagram schematically illustrating remote DMA.
FIG. 7 is a flowchart illustrating an example of processing (an information processing method) executed in the simulation system.
FIG. 8 is a diagram illustrating a schematic configuration of a modification.
FIG. 9 is a diagram schematically illustrating a flow of simulation.
FIG. 10 is a diagram illustrating a schematic configuration of a modification.
FIG. 11 is a diagram illustrating an example of a hardware configuration of an apparatus.

### Description of Embodiments

Embodiments of the present disclosure are explained in detail below with reference to the drawings. Note that, in the embodiments explained below, redundant explanation is omitted by denoting the same elements with the same reference numerals and signs.

The present disclosure is explained according to order of items described below.

### 0. Introduction

1. Embodiment
2. Modification
3. Example of a hardware configuration
4. Example of effects

### 0. Introduction

Surgery requires a high degree of skill of a doctor, and training is important. In the conventional surgical simulation, it is possible to perform video display or the like simulating an organ and learn a procedure for the organ. However, the surgical simulation does not accurately reflect deformation and a sense of force of an actual flexible organ is only used for initial training. Since it is difficult to handle a large-scale human body model, the surgical simulation is limited to a model simulation for a partial model organ.

For example, from the viewpoint of computing resources, it is difficult to handle a large-scale model including organs of an entire abdominal cavity. In order to perform precise simulation, it is necessary to make a mesh of a model denser. This also has a limit from the viewpoint of computing resources. In order to ensure computing resources, it is conceivable to use a high computing capability of cloud computing. However, there is a problem of communication delay. In particular, in the case of real-time simulation, the problem becomes apparent. It is also conceivable to realize sense-of-force estimation and feedback that match physical parameters such as a Young's modulus by combining an FEM (Finite Element Method). However, for more precise haptics control/sense-of-force feedback, it is necessary to shorten a period of an arithmetic operation for simulation and feedback.

In particular, since there is the problem of the delay explained above, simulation has been performed only by edge computing. Since sufficient computational capability cannot be obtained, model simplification and limitation of a modeling target portion have to be performed, and reality has been sacrificed. The period of the arithmetic operation needs to be set longer, and reality of sense-of-force feedback has also been sacrificed. At a long arithmetic operation period, it is difficult to realistically simulate deformation and the like of an organ to which the actual gravity is applied.

At least one of the problems explained above is addressed by the disclosed technique. By using the all-optical communication network, the problem of the low delay is addressed. Sufficient computing resources by cloud computing are ensured. By using the remote DMA (Direct Memory Access), it is possible to easily perform distributed processing across networks by synchronizing data present in memories of distributed information processing apparatuses in addition to low-delay data transfer.

By using high computational capability, for example, it is possible to perform a real-time simulation of deformation of a large-scale and flexible human organ model and sense-of-force calculation. It is possible to achieve both real-time processing of a large-scale human body model and real-time feedback of a precise sense-of-force. A simulation environment similar to actual surgery also based on a doctor's delicate sense is constructed. It can be expected that the quality of training of the doctor and preoperative planning is improved, leading to improvement of a surgical result.

### 1. Embodiment

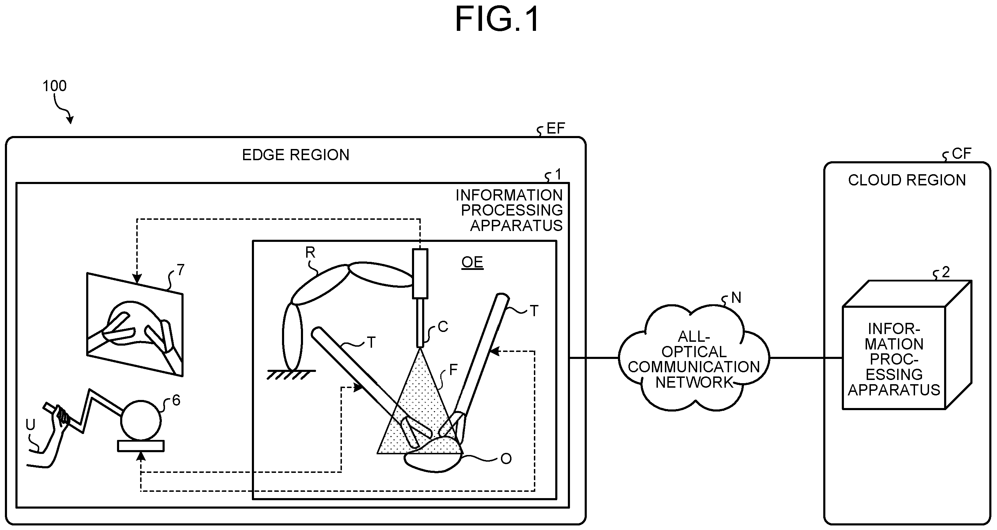

FIG. 1 is a diagram schematically illustrating an overview of a simulation system according to an embodiment. A simulation system 100 can be used as a haptic surgery simulator capable of performing a large-scale arithmetic operation. For example, the simulation system 100 can be used for training or preoperative planning of surgery for handling soft tissues (soft organs and the like) such as laparoscopic surgery, thoracic surgery, and brain surgery. By training while feeling deformation and real reaction of a soft tissue, more accurate prior experience can be performed and effects can be expected in improvement of skills and improvement of surgical results.

Note that the simulation system 100 can also be used as a training system of a surgical robot. Quite a few conventional surgical support robots do not have sense-of-force control. In this case, a doctor performs treatment while imagining force applied to an organ or a tissue only with a video. By calculating and presenting (for example, displaying) an accurate sense of- force, it is possible to grasp force applied to the organ and appropriate force at the time of suturing. More effective training can be performed.

In the example illustrated in FIG. 1, the simulation system 100 includes an information processing apparatus 1 and an information processing apparatus 2. The information processing apparatus 1 and the information processing apparatus 2 are a first information processing apparatus and a second information processing apparatus that provide simulation of surgery by cooperating with each other.

The information processing apparatus 1 and the information processing apparatus 2 are connected via an all-optical communication network N. As explained in detail below, the information processing apparatus 1 and the information processing apparatus 2 transmit and receive simulation data to and from each other via the all-optical communication network N.

The connection between the information processing apparatus 1 and the information processing apparatus 2 by the all-optical communication network N means that at least all communication between endpoint routers is constructed by optical communication. Note that communication after the endpoint routers may be electrical communication after photoelectric conversion. The information processing apparatus 1 and the information processing apparatus 2 are connected with a low delay by communication via the broadband all-optical communication network N. This is particularly useful for real-time simulation.

The information processing apparatus 1 is disposed in an edge region and configures an edge simulator terminal in conjunction with a haptic device 6 and a monitor 7. In this example, the haptic device 6 and the monitor 7 are also components of the information processing apparatus 1. However, the haptic device 6 and the monitor 7 may not be the components of the information processing apparatus 1. A user U of the simulation system 100 is located in an edge region EF. In FIG. 1, only the part of a hand of the user U operating the haptic device 6 is schematically illustrated.

A surgical environment OE is simulated by cooperation of the information processing apparatus 1 and the information processing apparatus 2. In FIG. 1, the surgical environment OE is schematically illustrated on the inner side of the information processing apparatus 1. The surgical environment OE includes virtual elements related to surgery. As elements included in the surgical environment OE, a soft tissue O, a surgical instrument T, a robot arm R, and a camera C are illustrated in FIG. 1.

Examples of the soft tissue O include an organ having flexibility. An example of the surgical instrument T is a surgical instrument such as forceps. Unlike the soft tissue O, the surgical instrument T may have rigidity. The robot arm R is a surgical robot arm that supports a camera C. The camera C is a camera for surgery and images a surgical field including the soft tissue O and the surgical instrument T. A field of view of the camera C is referred to as field of view F and illustrated.

The haptic device 6 (a tactile device) is a device that the user U touches (with a hand) and operates in order to operate the surgical instrument T. The user U moves the haptic device 6 to operate the surgical instrument T. By holding and cutting the soft tissue O using the surgical instrument T, the surgical simulation is advanced. When the surgical instrument T comes into contact with the soft tissue O, an external force is applied to the soft tissue O and the soft tissue O is simulated to be, for example, deformed. A contact force (which can be equivalent to, for example, a sense of force) generated by interaction between the soft tissue O and the surgical instrument T is reflected in the operation of the surgical instrument T by the user U via the haptic device 6 and fed back to the user U.

The monitor 7 displays a video in a field of view F of the camera C explained above, that is, a video including (at least a part of) the soft tissue O and the surgical instrument T. The user U operates the haptic device 6 while viewing the video displayed on the monitor 7. The position, the angle, and the like of the camera C may also be operated by the user U. An operation unit of the camera C may be incorporated in the haptic device 6 or may be provided separately from the haptic device 6. Operation of the camera C may be automatically performed. For example, a function of a control unit that automatically operates the camera C may be incorporated in the surgical environment OE.

The information processing apparatus 2 is disposed in a cloud region CF. The cloud region CF is a region located on the opposite side to the edge region EF across the all-optical communication network N and is, for example, a region away from the edge region EF.

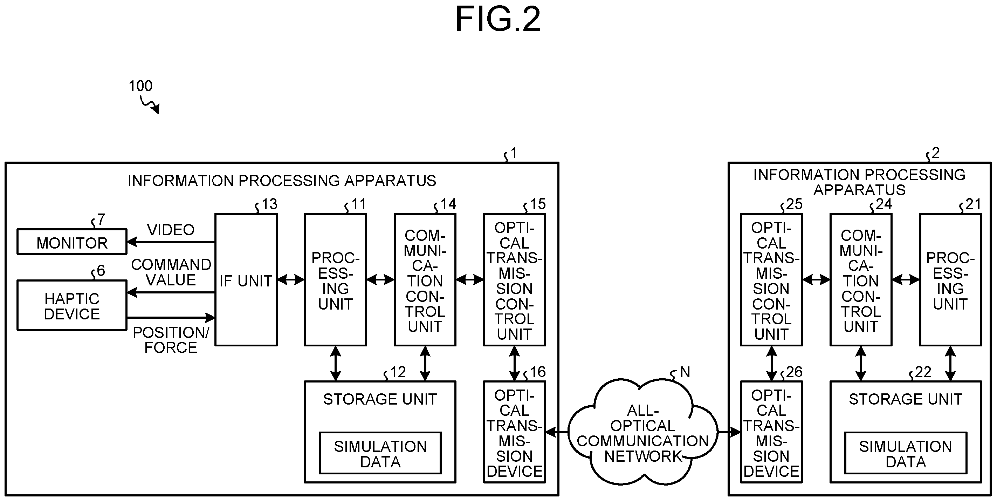

A more specific configuration of the simulation system 100 is explained with reference to FIG. 2.

FIG. 2 is a diagram illustrating an example of functional blocks of the simulation system. In FIG. 2, an example of functional blocks of the information processing apparatus 1 and the information processing apparatus 2 is illustrated.

Besides the haptic device 6 and the monitor 7 explained above, the information processing apparatus 1 includes a processing unit 11, a storage unit 12, an IF unit 13, a communication control unit 14, an optical transmission control unit 15, and an optical transmission device 16.

The processing unit 11 functions as an overall control unit (a main control unit) that controls the elements of the information processing apparatus 1. The processing unit 11 executes processing concerning simulation. Details are explained below.

The storage unit 12 stores information used in the information processing apparatus 1. As the information stored in the storage unit 12, simulation data is exemplified. The simulation data includes data used in an arithmetic operation of simulation and the like and data obtained by the simulation.

The IF unit 13 provides an interface between the processing unit 11 and the haptic device 6 and the monitor 7. Information (a motion) concerning a position and force is input from the haptic device 6 to the processing unit 11 via the IF unit 13. The motion may also include information such as a posture. The processing unit 11 causes a motion of the robot arm R in the surgical environment OE to correspond to the motion input via the haptic device 6. The user U operates the surgical instrument T in the surgical environment OE via the haptic device 6.

A contact force generated by contact of the surgical instrument T and the soft tissue O is calculated. The calculated contact force is fed back to the user U via the haptic device 6. A command value for reflecting the contact force on the haptic device 6 is sent from the processing unit 11 to the haptic device 6 via the IF unit 13.

As explained above, the surgical environment OE includes the camera C and a video of the camera C is displayed by the monitor 7 (FIG. 1). The video of the camera C is sent from the processing unit 11 to the monitor 7 via the IF unit 13 and displayed.

The communication control unit 14 controls communication between the information processing apparatus 1 and an external apparatus. An example of the external apparatus is the information processing apparatus 2. In that case, an optical transmission control unit 15 and an optical transmission device 16 explained below are used.

The optical transmission control unit 15 controls optical transmission by the optical transmission device 16. The optical transmission device 16 is a device for connecting the information processing apparatus 1 to the all-optical communication network N. The optical transmission device 16 is mounted on, for example, a PCIe (Peripheral Component Interconnect Express) or the like and is incorporated in the information processing apparatus 1.

The information processing apparatus 2 includes a processing unit 21, a storage unit 22, a communication control unit 24, an optical transmission control unit 25, and an optical transmission device 26.

The processing unit 21 functions as an overall control unit that controls the elements of the information processing apparatus 2. The processing unit 21 executes processing concerning simulation. Details are explained below.

The storage unit 22 stores information used in the information processing apparatus 2. As the information stored in the storage unit 22, simulation data is exemplified.

The communication control unit 24 controls communication between the information processing apparatus 1 and an external apparatus. An example of the external apparatus is the information processing apparatus 1. In that case, the optical transmission control unit 25 and the optical transmission device 26 are used.

The optical transmission control unit 25 controls optical transmission by the optical transmission device 26. The optical transmission device 26 is a device for connecting the information processing apparatus 2 to the all-optical communication network N.

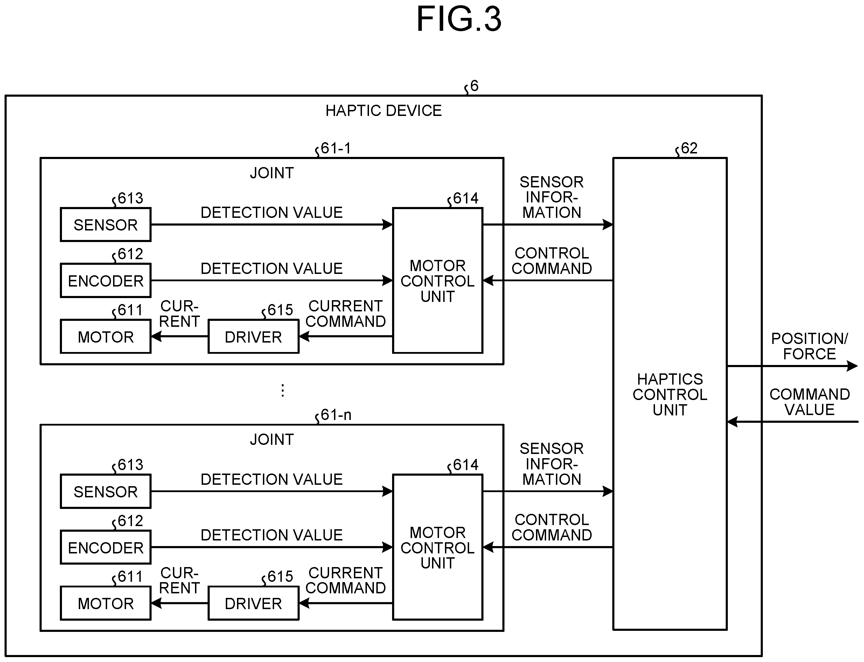

A specific configuration of the haptic device 6 is explained with reference to FIG. 3.

FIG. 3 is a block diagram illustrating an example of a schematic configuration of the haptic device. The haptic device 6 includes a plurality of (n) joints 61 and a haptics control unit 62. The joints 61 are referred to as a joint 61-1 and a joint 61-n and illustrated to be distinguishable from each other.

The joint 61 includes a motor 611, an encoder 612, a sensor 613, a motor control unit 614, and a driver 615. The motor 611 is rotated according to an electric current driven by the driver 615. The encoder 612 detects a rotational position of the joint 61. Examples of the sensor 613 are a force sensor, a torque sensor, an acceleration sensor, and the like. The sensor 613 detects a force and torque applied to the joint 61, acceleration of the joint 61, and the like. The sensor may be a six-axis sensor or a plurality of sensors may be used in combination as the sensor. It is possible to perform more precise force control.

The motor control unit 614 feedback-controls the motor 611 based on a detection value of the encoder 612 and a detection value of the sensor 613. For example, the position, the force, the torque, the acceleration, and the like of the joint 61 are controlled. The feedback control may include an arithmetic operation of force control and acceleration control including estimation of a disturbance observer. The motor 611 is controlled via the driver 615. The motor control unit 614 gives a current command to the driver 615. The driver 615 drives an electric current of the motor 611 based on the given current command.

The motor control unit 614 sends sensor information, for example, a detection value of the encoder 612, a detection value of the sensor 613, and the like to the haptics control unit 62.

The haptics control unit 62 sends position/force information to the processing unit 11 (FIG. 2) based on sensor information from the motor control units 614 of the joints 61.

The haptics control unit 62 sends a control command to the motor control units 614 of the joints 61 based on a command value from the processing unit 11 (FIG. 2). The motor control units 614 of the joints 61 control the motor 611 based on the control command from the haptics control unit 62.

The simulation system 100 is further explained with reference to FIG. 2 again. In the simulation system 100, processing required for simulation is shared and borne by the processing unit 11 of the information processing apparatus 1 and the processing unit 21 of the information processing apparatus 2.

The processing unit 21 of the information processing apparatus 2 disposed in the cloud region CF has higher calculation capability than the processing unit 11 of the information processing apparatus 1 disposed in the edge region EF. Among kinds of processing required for the simulation, processing with a particularly large calculation load is executed by the processing unit 21 of the information processing apparatus 2. An example of the processing is explained.

The processing unit 11 of the information processing apparatus 1 simulates a motion of the surgical instrument T. The position/force information is input from the haptic device 6 to the processing unit 11. Since the surgical instrument T has rigidity and is not deformed unlike the soft tissue O, a calculation load is not so large.

The processing unit 21 of the information processing apparatus 2 calculates a simulation model including the soft tissue O. The calculation of the simulation model includes modeling of the soft tissue O and calculation of deformation of the soft tissue O. For example, FEM, a material point method (MPM), or the like may be used. Although the calculation load is large, since the processing unit 21 of the information processing apparatus 2 has high calculation capability, it is possible to perform calculation of a highly accurate simulation model at high speed.

In the simulation of the soft tissue O, an XPBD (Extended Position Based Dynamics) method may be used in combination with the FEM. For example, deformation of the soft tissue O to which an external force is applied is calculated, with conditions for minimizing the potential energy density of the polyhedron modeled based on the FEM as constraints, from the distance among the plurality of particles in the polyhedron and the mass of the plurality of particles. The constraints are equivalent to constraints in the XPBD.

The processing unit 21 of the information processing apparatus 2 calculates a contact force generated by interaction between the soft tissue O and the surgical instrument T. The contact force is calculated, for example, by simulating contact between the soft tissue O and the surgical instrument T after deformation. highly accurate contact force calculation based on a highly accurate deformation calculation result of the soft tissue O is possible. Note that, in addition to the contact force, pressure (holding force) or the like applied to an opening and closing/gripping portion at the distal end of the surgical instrument T may be calculated. In a range without contradiction, the pressure explained above may also be understood to be included in the contact force.

By using the high calculation capability of the processing unit 21 of the information processing apparatus 2, it is possible to reduce a calculation period (an arithmetic operation period) of the simulation, in other words, it is possible to increase a frame frequency of the simulation. Specifically, the processing unit 21 calculates deformation of a model, presence or absence of collision, a contact force, gravity, and the like at a period that is shorter than a calculation period in the case in which the information processing apparatus 1 is assumed to have performed the calculation thereof and is the same as or longer than a control period of the haptic device 6. For example, the processing unit 21 calculates a simulation model at a predetermined period higher than the conventional drawing processing and equivalent to motion control of a surgical robot. An example of the period explained above is a period equal to or higher than 60 Hz and approximately the same as (for example, substantially equal to) 1 kHz.

The processing unit 11 of the information processing apparatus 1 controls the haptic device 6 such that the contact force calculated by the processing unit 21 of the information processing apparatus 2 is fed back to the user U (haptics control processing). A control period of the haptic device 6 may be the same as the period of the calculation of the simulation model by the processing unit 21 of the information processing apparatus 2. For example, by controlling the haptic device 6 at a fast period (a short period) of 1 kHz or more, it is possible to perform more precise haptic feedback than when the haptic device 6 is controlled at a slow period (a long period) of approximately 60 Hz. Haptics feedback corresponding to sagging or the like of an organ considering the gravity is also possible.

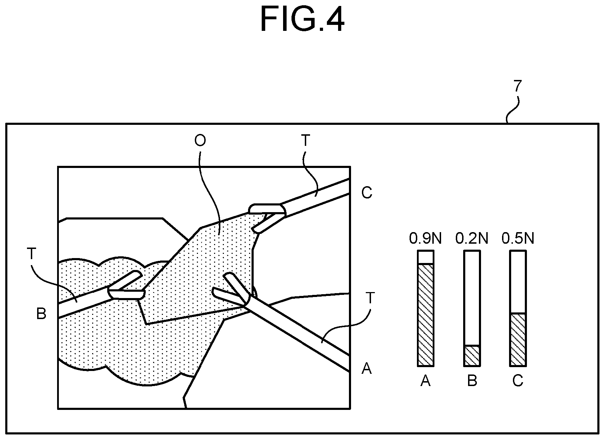

The processing unit 11 of the information processing apparatus 1 generates a video including the soft tissue O simulated by the processing unit 21 of the information processing apparatus 2 (drawing processing). An example of the period of the drawing processing is 60 Hz or the like. The drawing processing may include CG rendering processing. Ray tracing may be used. It is possible to generate a photorealistic video. The processing unit 11 may generate a video such that information concerning the contact force calculated by the processing unit 21 of the information processing apparatus 2 is included in the video. This is explained with reference to FIG. 4.

FIG. 4 is a diagram illustrating an example of the video. The soft tissue O and the surgical instrument T are displayed in a left side portion of the monitor 7. Three surgical instruments T are exemplified. Alphabets A, B, and C for distinguishing the surgical instruments T are displayed in association with one another. In a right side portion of the monitor 7, information concerning contact forces corresponding to the surgical instruments T, specifically, values (a unit is N) of the contact forces are displayed as numerical values and bars.

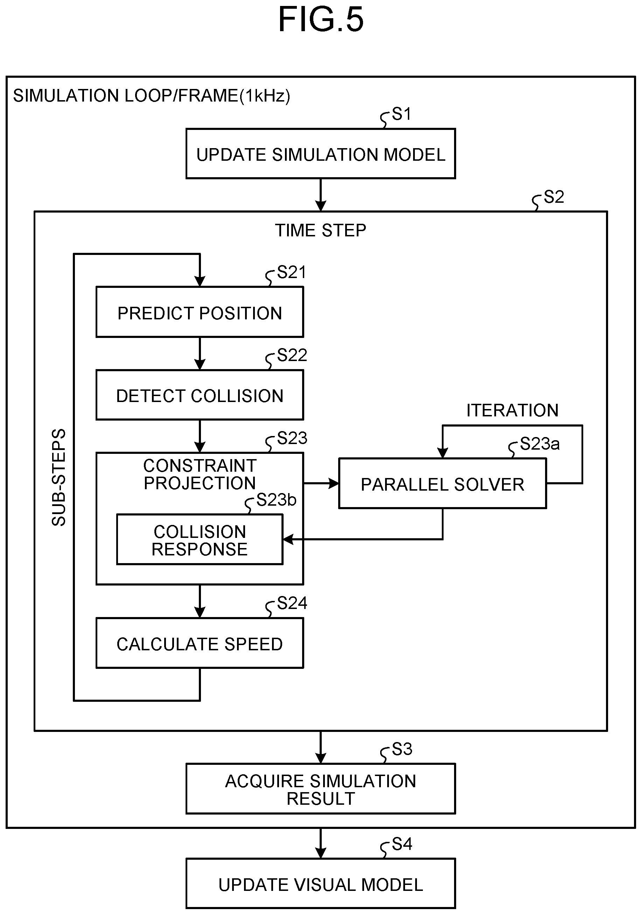

FIG. 5 is a diagram illustrating an example of a simulation loop. Processing executed during one frame is schematically illustrated. In this example, the XPBD method explained above is used for simulation of the soft tissue O.

In step S1, the simulation model is updated. For example, the soft tissue O is dissected, mesh topology changes, and a model of the soft tissue O or the like is updated.

In step S2, simulation using the soft tissue O or the like updated in the preceding step S1 is executed. The processing in step S2 is subdivided into a fixed number of sub-steps. In the example illustrated in FIG. 5, the processing in sub-step S21 to sub-step S24 is repeatedly executed.

In sub-step S21, the positions of meshed portions are predicted. In sub-step S22, collision corresponding to the predicted positions is detected. In sub-step S23, so-called constraint projection is performed, the FEM-based constraints are solved based on the XPBD, and a new position is calculated in parallel (sub-step S23a). In this example, iterative processing by the Jacobi method is executed. A calculation result is obtained as a collision response (sub-step S23b).

In sub-step S24, speed is calculated and updated based on the collision response, that is, a new position. After the processing of the sub-step S24, the processing in sub-step S21 is executed again. This sub-step loop is repeated within a range of the time step in step S2.

In step S3, the simulation result in the preceding step S2 is acquired. In this processing, the simulation result includes the soft tissue O after deformation.

In step S4, the visual model is updated and reflected in video display.

For example, the above processing is repeatedly executed at a fast period of 1 kHz or more.

Referring back to FIG. 2, necessary simulation data is transmitted and received between the information processing apparatus 1 and the information processing apparatus 2. The simulation data is transmitted and received via the all-optical communication network N. By using the all-optical communication network N, a delay in the transmission and reception of the simulation data can be further suppressed (a delay can be further reduced) than when the all-optical communication network N is not used.

With the simulation system 100, by using the high calculation capability of the processing unit 21 of the information processing apparatus 2, it is possible to perform calculation of a large-scale simulation model, realization of which is difficult only by the processing unit 11 of the information processing apparatus 1. Calculation of a model of the entire abdominal cavity or the like is also possible. Deformation and the like of the soft tissue O can be accurately calculated. Since the calculation capability is high, a fine mesh model can be used. Therefore, deformation and the like of the small soft tissue O can be accurately calculated. Simulation of the real surgical environment OE can be provided.

By using the all-optical communication network N, the simulation data can be transmitted and received with a low delay. Simulation having real time properties can also be provided.

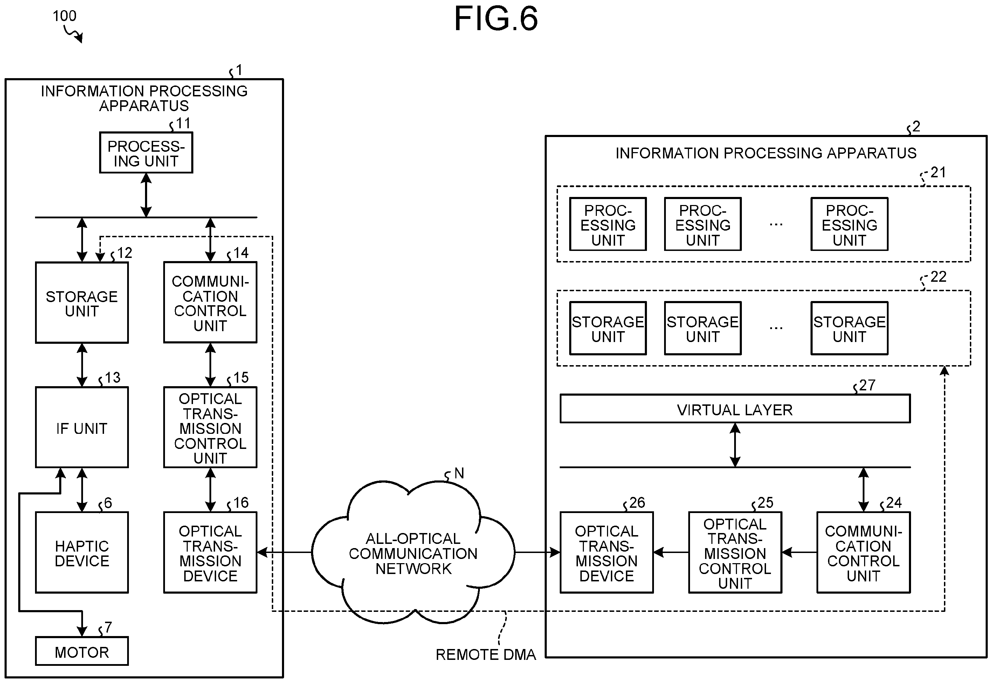

In one embodiment, the information processing apparatus 1 and the information processing apparatus 2 may transmit and receive the simulation data to and from each other with remote DMA. In this case, update-processed data is transmitted and received between the storage unit 12 of the information processing apparatus 1 and the storage unit 22 of the information processing apparatus 2 by the remote DMA without intervention of the CPU and is synchronized. By using the remote DMA, it is possible to further reduce the delay. This is explained with reference to FIG. 6.

FIG. 6 is a diagram schematically illustrating the remote DMA. In this example, in the information processing apparatus 2, the functions of the processing unit 21 and the storage unit 22 are realized on a virtual layer 27.

(At least a part of) the simulation data stored in the storage unit 12 of the information processing apparatus 1 and (at least a part of) the simulation data stored in the storage unit 22 of the information processing apparatus 2 can be synchronized by a path of the remote DMA indicated by a broken line arrow. For example, motion data or the like of the surgical instrument T processed by the processing unit 11 of the information processing apparatus 1 and data or the like of a simulation model processed by the processing unit 21 of the information processing apparatus 2 are shared with a low delay. The effect of accelerating the simulation period can be further enhanced.

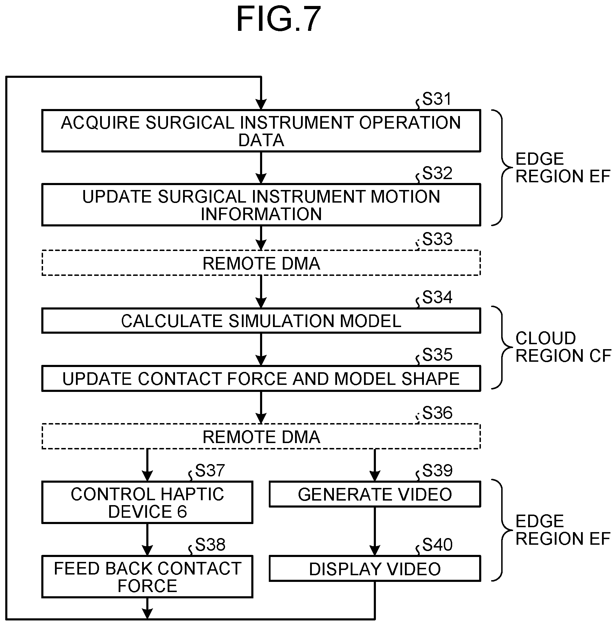

FIG. 7 is a flowchart illustrating an example of processing (an information processing method) executed in the simulation system. Processing in steps S31, S32, and S37 to S40 is executed in the edge region EF. Processing in steps S34 and S35 is executed in the cloud region CF. Processing in steps S33 and S36 is the remote DMA.

In step S31, surgical instrument operation data is acquired. The user U operates the haptic device 6. The haptic device 6 acquires surgical instrument operation data corresponding to the user operation. Position/force Information corresponding to the surgical instrument operation data is transmitted from the haptic device 6 to the processing unit 11 of the information processing apparatus 1.

In step S32, surgical instrument motion information is updated. The processing unit 11 of the information processing apparatus 1 updates a motion of the surgical instrument T in the surgical environment OE.

In step S33, the remote DMA is performed. Simulation data in the storage unit 12 of the information processing apparatus 1 is transferred to the storage unit 22 of the information processing apparatus 2.

In step S34, a simulation model is calculated. The processing unit 21 of the information processing apparatus 2 calculates deformation of the soft tissue O and calculates a contact force.

In step S35, a contact force and a model shape are updated. The processing unit 21 of the information processing apparatus 2 updates the simulation model such as the contact force and the shape of the soft tissue O based on a calculation result of the simulation model.

In step S36, the remote DMA is performed. Simulation data in the storage unit 22 of the information processing apparatus 2 is transferred to the storage unit 12 of the information processing apparatus 1. After the processing of step S36, the processing is advanced to each of step S37 and step S39.

In step S37, the haptic device 6 is controlled. The processing unit 11 of the information processing apparatus 1 controls the haptic device 6 such that the updated contact force is fed back to the user U.

In step S38, the contact force is fed back. Through the haptic device 6, the contact force is fed back (a sense of force is presented) to the user U.

In step S39, a video is generated. The processing unit 11 of the information processing apparatus 1 generates a video including the updated soft tissue O and the like.

In step S40, the video is displayed. The monitor 7 displays the video.

When the processing in step S38 and step S40 is completed, the processing is returned to step S31. A series of processing is repeatedly executed and the simulation is advanced.

### 2. Modification

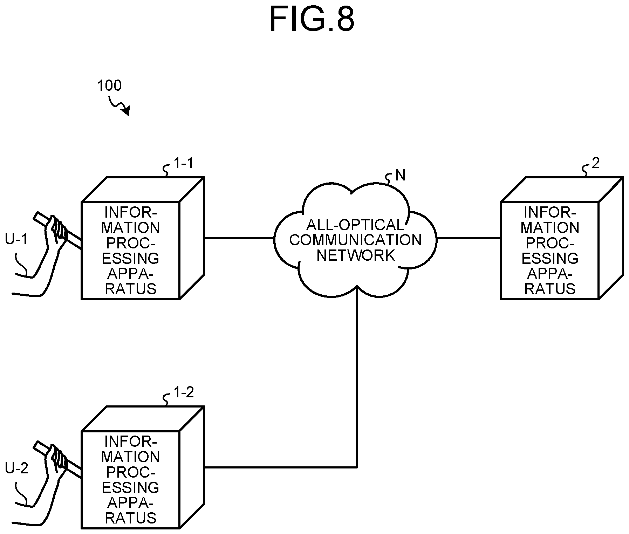

The disclosed techniques are not limited to the embodiment explained above. Some modifications are explained. For example, the simulation system 100 may include a plurality of information processing apparatuses 1 respectively used by different users U. Multi-base connection via the all-optical communication network N is possible. This is explained with reference to FIG. 8.

FIG. 8 is a diagram illustrating a schematic configuration of a modification. An information processing apparatus 1-1 and an information processing apparatus 1-2 are exemplified as the plurality of information processing apparatuses 1. The information processing apparatus 1-1 and the information processing apparatus 1-2 may be disposed apart from each other or may be disposed close to each other.

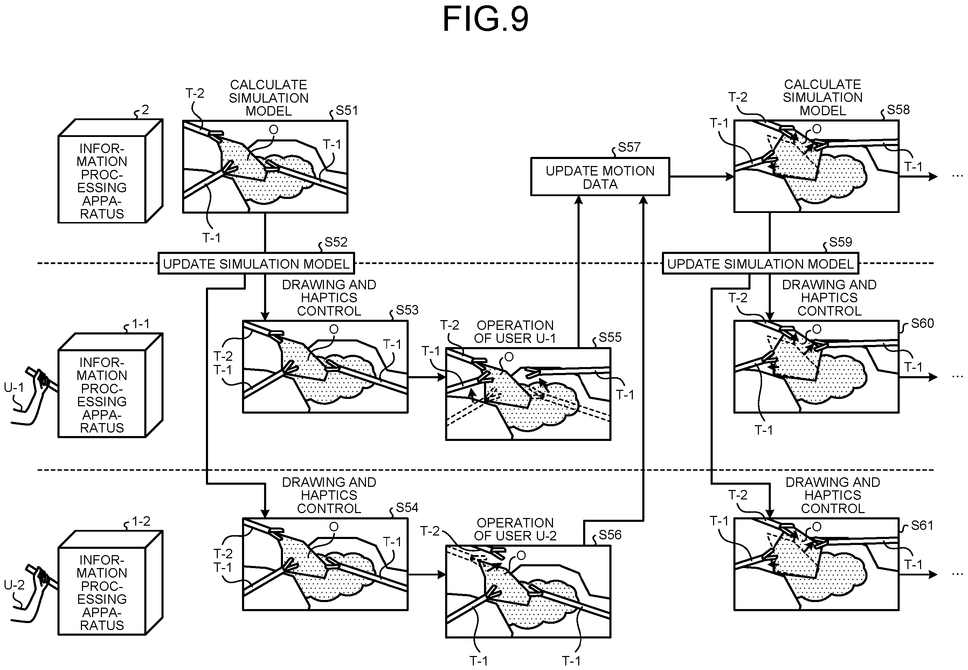

The user U who operates (the haptic device 6 of) the information processing apparatus 1-1 is referred to as user U-1 and illustrated. The user U who operates (the haptic device 6 of) the information processing apparatus 1-2 is referred to as user U-2 and illustrated. For example, the user U-1 is a surgeon and the user U-2 is an assistant. Operations of both of the user U-1 and the user U-2 is reflected in simulation. For example, the user U-1 and the user U-2 operate respective surgical instruments to treat the same soft tissue O. This is explained with reference to FIG. 9.

FIG. 9 is a diagram schematically illustrating a flow of simulation. The surgical instrument T operated by the user U-1 is referred to as surgical instrument T-1 and illustrated. The surgical instrument T operated by the user U-2 is referred to as surgical instrument T-2 and illustrated.

In step S51, a simulation model is calculated. The processing unit 21 of the information processing apparatus 2 calculates a simulation model based on a motion of the surgical instrument T-1 and a motion of the surgical instrument T-2. In step S52, the simulation model is updated. Updated data is transferred to the information processing apparatus 1-1 and the information processing apparatus 1-2.

In step S53, drawing and haptics control by the information processing apparatus 1-1 is performed. The processing unit 11 of the information processing apparatus 1-1 generates a video based on the updated simulation model and controls the haptic device 6.

In step S54, drawing and haptics control by the information processing apparatus 1-2 is performed. The processing unit 11 of the information processing apparatus 1-1 generates a video based on the updated simulation model and controls the haptic device 6.

In step S55, operation by the user U-1 is performed. The processing unit 11 of the information processing apparatus 1-1 simulates a motion of the surgical instrument T-1 according to operation of the haptic device 6 by the user U-1. As exemplified by an arrow extending from a broken line, the motion of the surgical instrument T-1 changes. Motion data of the surgical instrument T-1 is transferred to the information processing apparatus 2.

In step S56, operation by the user U-2 is performed. The processing unit 11 of the information processing apparatus 1-2 simulates a motion of the surgical instrument T-2 according to operation of the haptic device 6 by the user U-2. As exemplified by an arrow extending from a broken line, the motion of the surgical instrument T-2 changes. Motion data of the surgical instrument T-2 is transferred to the information processing apparatus 2.

In step S57, the motion data of the surgical instrument T on the information processing apparatus 2 side is updated.

The respective kinds of processing are repeatedly executed with the processing from step S51 to step S57 as one cycle. Since processing in step S58 to step S61 illustrated in FIG. 9 is similar to the processing in step S52 to step S54, the explanation is not repeated.

For example, as explained above, a plurality of users U can treat the same soft tissue O using different surgical instruments T. It is possible to perform surgery training by the plurality of users U.

The simulation explained above may be applied to a surgical robot simulation. In the surgical robot simulation, the user U operates, via the haptic device 6, the robot arm supporting a surgical instrument T. Since the contact force is fed back to the user U, it is also possible to evaluate, for example, whether the robot arm and the organ can be operated with an appropriate operation force and whether an excessive force is not applied, whereby safe operation of the robot can be learned.

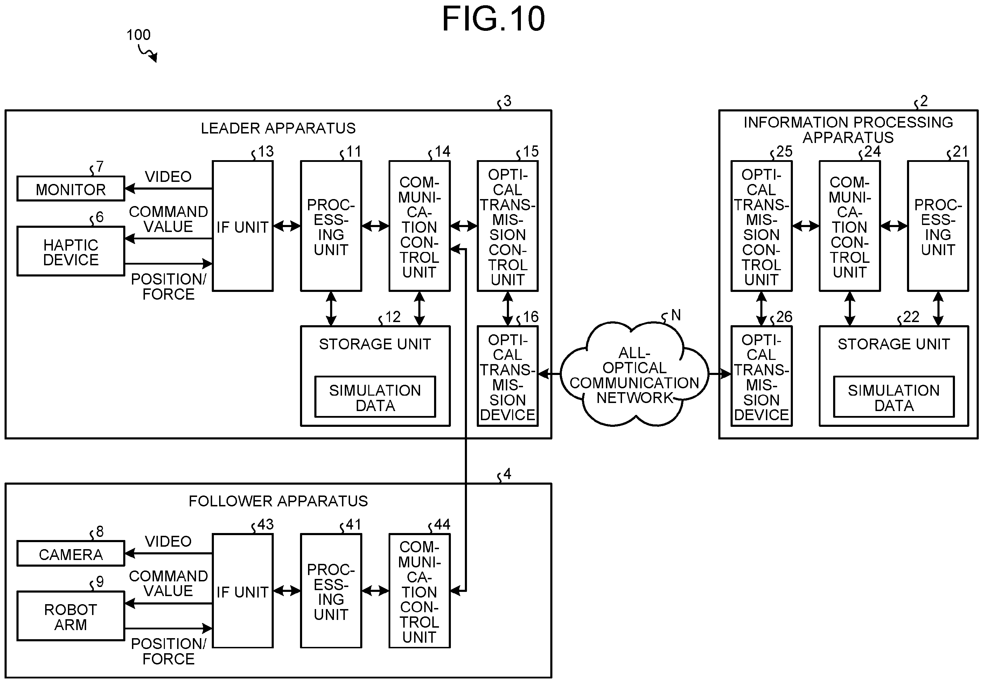

In one embodiment, the simulation may be incorporated into a master slave system. The embodiment is explained with reference to FIG. 10.

FIG. 10 is a diagram illustrating a schematic configuration of a modification. The simulation system 100 includes a leader apparatus 3, a follower apparatus 4, and the information processing apparatus 2.

Basic components of the leader apparatus 3 are the same as the components of the information processing apparatus 1 explained above. The communication control unit 14 of the leader apparatus 3 also controls communication between the leader apparatus 3 and the follower apparatus 4.

The follower apparatus 4 includes a camera 8, a robot arm 9, a processing unit 41, an IF unit 43, and a communication control unit 44. The camera 8 captures an image of a surgical field and the robot arm 9 supports the surgical instrument T.

The processing unit 41 functions as an overall control unit that controls elements of the follower apparatus 4. The processing unit 41 observes a slave environment including a state of a patient based on a video of the camera 8. The slave environment may also include non-visual information such as a blood pressure and a heart rate of the patient. The processing unit 41 controls the robot arm 9 and switches operation of the position of the camera 8 according to operation of the haptic device 6 in the leader apparatus 3.

The IF unit 43 provides an interface between the processing unit 41 and the camera 8 and the robot arm 9.

The communication control unit 44 controls communication between the follower apparatus 4 and the leader apparatus 3. Note that communication between the follower apparatus 4 and the information processing apparatus 2 may also be controlled.

The leader apparatus 3 and the follower apparatus 4 function as a master apparatus and a slave apparatus capable of performing bilateral control. Surgery is advanced by controlling, with a master-slave scheme, the robot arm 9 supporting the surgical instrument T. A video of the camera 8 of the follower apparatus 4 is transmitted from the follower apparatus 4 to the leader apparatus 3 and is displayed on the monitor 7 of the leader apparatus 3. Information concerning other slave environments is also transmitted from the follower apparatus 4 to the leader apparatus 3 and presented. Position/force Information from the haptic device 6 of the leader apparatus 3 is transmitted from the leader apparatus 3 to the follower apparatus 4 and is reflected in control of the robot arm 9. Position/force information from the robot arm 9 is transmitted from the follower apparatus 4 to the leader apparatus 3 and is fed back to the user via the haptic device 6 of the leader apparatus 3.

In the information processing apparatus 2, the highly accurate simulation model explained above is calculated and updated. In the simulation system 100 illustrated in FIG. 10, the result of the simulation is reflected in the control of the robot arm 9. For example, when the position/force information from the haptic device 6 of the leader apparatus 3 is transmitted to the follower apparatus 4, the control of the robot arm 9 is limited in order to ensure safety.

Specifically, the processing unit 21 of the information processing apparatus 2 executes safety determination processing based on a calculation result of the simulation model. For example, the processing unit 21 determines whether operation of the robot arm 9 of the follower apparatus 4 via the leader apparatus 3 is dangerous. When determining that the operation is dangerous, the processing unit 21 intervenes in the operation of the follower apparatus 4 by the leader apparatus 3. For example, the processing unit 21 corrects the position/force information transmitted from the leader apparatus 3 to the follower apparatus 4 such that a movement amount of the robot arm 9 is limited and operation force is limited. The corrected position/force information is transmitted from the leader apparatus 3 to the follower apparatus 4. A determination result and a control result by the processing unit 21 of the information processing apparatus 2 are notified to the leader apparatus 3 and the follower apparatus 4. Note that, in the safety monitoring explained above, a state slightly in the future from the current time may be simulated while a future motion being predicted based on movement data of the haptic device 6. By using the high calculation capability of the information processing apparatus 2, the simulation explained above can be performed in real time. The safety can be further improved.

### 3. Example of a hardware configuration

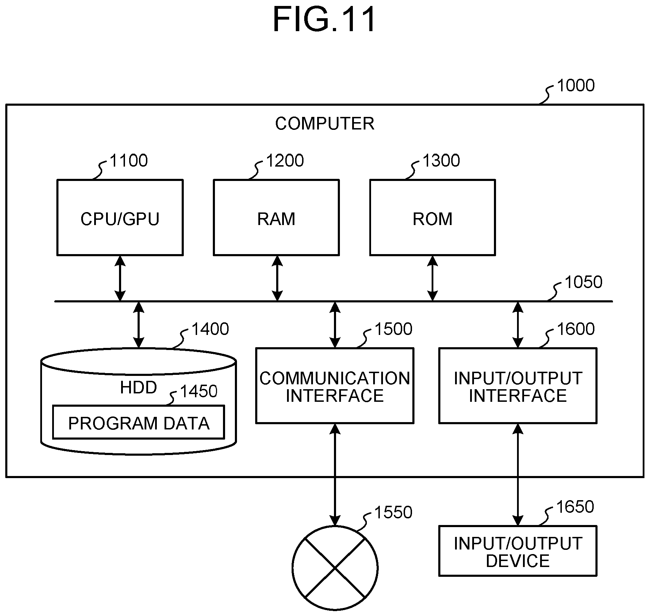

FIG. 11 is a diagram illustrating an example of a hardware configuration of an apparatus. The information processing apparatus 1, the information processing apparatus 2, and the like explained above are implemented by, for example, the computer 1000 illustrated in FIG. 11.

The computer 1000 includes a CPU/GPU 1100, a RAM 1200, a ROM (Read Only Memory) 1300, an HDD (Hard Disk Drive) 1400, a communication interface 1500, and an input/output interface 1600. The units of the computer 1000 are connected by a bus 1050.

The CPU/GPU 1100 operates based on a program stored in the ROM 1300 or the HDD 1400 and controls the units. For example, the CPU/GPU 1100 loads, in the RAM 1200, programs stored in the ROM 1300 or the HDD 1400 and executes processing corresponding to the various programs.

The ROM 1300 stores a boot program such as a BIOS (Basic Input Output System) to be executed by the CPU/GPU 1100 when the computer 1000 is started, a program depending on hardware of the computer 1000, and the like.

The HDD 1400 is a computer-readable recording medium that non-transiently records a program to be executed by the CPU/GPU 1100, data used by the program, and the like. Specifically, the HDD 1400 is a recording medium that records a program for an information processing method according to the present disclosure that is an example of the program data 1450.

The communication interface 1500 is an interface for the computer 1000 to be connected to an external network 1550 (for example, the Internet). For example, the CPU/GPU 1100 receives data from other equipment or transmits data generated by the CPU/GPU 1100 to the other equipment via the communication interface 1500.

The input/output interface 1600 is an interface for connecting an input/output device 1650 and the computer 1000. For example, the CPU/GPU 1100 receives data from an input device such as a keyboard or a mouse via the input/output interface 1600. The CPU/GPU 1100 transmits data to an output device such as a display, a speaker, or a printer via the input/output interface 1600. When the apparatus is the information processing apparatus 1, the CPU/GPU 1100 receives motion data from the haptic device 6 or transmits a simulated contact force to the haptic device via the input/output interface 1600. The input/output interface 1600 may function as a media interface that reads a program or the like recorded in a computer-readable predetermined recording medium (a medium). The medium is, for example, an optical recording medium such as a DVD (Digital Versatile Disc) or a PD (Phase change rewritable Disk), a magneto-optical recording medium such as a MO (Magneto-Optical Disk), a tape medium, a magnetic recording medium, or a semiconductor memory.

When the computer 1000 functions as the information processing apparatus 1 and the information processing apparatus 2 explained above, the CPU/GPU 1100 of the computer 1000 executes a program loaded on the RAM 1200 to implement the functions of the processing unit 11 and the processing unit 21. The program may be stored in the HDD 1400. Note that the CPU/GPU 1100 reads the program data 1450 from the HDD 1400 and executes the program data but, as another example, may acquire the program from another apparatus via the external network 1550.

The components explained above may be configured using general-purpose members or may be configured by hardware specialized for the functions of the components. The configuration explained above can be changed as appropriate according to a technical level at each time when the configuration is implemented.

### 4. Example of effects

The techniques explained above are specified, for example, as explained below. One of the disclosed techniques is the simulation system 100. As explained with reference to FIG. 1, FIG. 2, and the like, the simulation system 100 includes the information processing apparatus 1 (the first information processing apparatus) and the information processing apparatus 2 (the second information processing apparatus) that provide simulation of surgery in cooperation with each other. The information processing apparatus 1 and the information processing apparatus 2 transmit and receive simulation data to and from each other via the all-optical communication network N.

With the simulation system 100 explained above, simulation is provided by cooperation of the information processing apparatus 1 and the information processing apparatus 2. By using the all-optical communication network N, a delay in communication between the information processing apparatus 1 and the information processing apparatus 2 can be suppressed (can be reduced). Therefore, for example, simulation performance can be further improved than when simulation is provided only by the information processing apparatus 1. In particular, the simulation system 100 is useful for simulation having a function such as haptics presentation requiring real-time properties and, for example, can more highly accurately perform simulation such as deformation of a flexible object or a fine tissue.

As explained with reference to FIG. 6 and the like, the information processing apparatus 1 and the information processing apparatus 2 may transmit and receive simulation data to and from each other with the remote DMA. This makes it possible to further reduce the delay.

As explained with reference to FIG. 1, FIG. 2, FIG. 5, and the like, the information processing apparatus 2 may calculate a simulation model including the soft tissue O (for example, an organ). The calculation of the simulation model may include calculation of deformation of the soft tissue O. The information processing apparatus 2 may calculate a contact force generated by interaction between the soft tissue O and the surgical instrument T. For example, simulation performance can be improved by the information processing apparatus 2 executing the processing with a large calculation load explained above. It is also possible to perform a fine simulation at a period higher in speed than a period used in general video processing and suitable for calculation of a sense of force, for example, 1 kHz or more.

As explained with reference to FIG. 1 to FIG. 3 and the like, the information processing apparatus 1 may control the haptic device 6 operated by the user U such that the contact force calculated by the information processing apparatus 2 is fed back to the user U. The information processing apparatus 2 may calculate a contact force at a predetermined period (further calculate deformation of a model, presence or absence of collision, gravity, and the like). The information processing apparatus 1 may controls the haptic device 6 such that the contact force calculated by the information processing apparatus 2 is fed back to the user U at the period. The predetermined period may be shorter than a calculation period in the case in which it is assumed that the information processing apparatus 1 has performed calculation of a simulation model including calculation of a contact force and may be the same as or longer than a control period of the haptic device 6. The predetermined period may be a period of 60 Hz or more and approximately the same as 1 kHz. It is possible to perform accurate haptics feedback.

The haptic device 6 may include the joint 61 in which at least one of acceleration and force is controlled. The haptic device 6 may include the joint 61 in which at least one of a six-axis force sensor and a torque sensor is provided. By using the haptic device 6 explained above, it is possible to perform more precise haptic feedback.

As explained with reference to FIG. 8 and the like, the information processing apparatus 1 may be a plurality of information processing apparatuses 1 (for example, the information processing apparatus 1-1 and the information processing apparatus 1-2) used by different users U (for example, the user U-1 and the user U-2). It is possible to perform simulation of surgery by a plurality of users U.

As explained with reference to FIG. 10 and the like, the surgery may be surgery for controlling, with by the master-slave scheme, the robot arm 9 supporting the surgical instrument T and a result of the simulation may be reflected in control of the robot arm 9. For example, as explained above, the simulation can be incorporated in the master slave system and used.

The information processing apparatus 2 explained with reference to FIG. 1, FIG. 2, and the like is also one of the disclosed techniques. The information processing apparatus 2 provides simulation of surgery in cooperation with the information processing apparatus 1 (another information processing apparatus). The information processing apparatus 2 includes the processing unit 21 that calculates a simulation model including the soft tissue O and an optical transmission control unit that controls transmission and reception of simulation data to and from the information processing apparatus 1 via the all-optical communication network N. Such an information processing apparatus 2 can also improve the simulation performance as explained above.

The information processing method explained with reference to FIG. 7 and the like is also one of the disclosed techniques. In the information processing method, the information processing apparatus 1 (the first information processing apparatus) and the information processing apparatus 2 (the second information processing apparatus) cooperate with each other to provide simulation of surgery. The information processing method includes the information processing apparatus 1 and the information processing apparatus 2 transmitting and receiving simulation data to and from each other via the all-optical communication network N (step S33 and step S36). With the information processing method explained above as well, the simulation performance can be improved as explained above.

Note that the effects described in the present disclosure are only exemplifications and are not limited by the disclosed content. There may be other effects.

Although the embodiment of the present disclosure is explained above, the technical scope of the present disclosure is not limited to the embodiment explained above per se. Various changes are possible without departing from the gist of the present disclosure. Components in different embodiments and modifications may be combined as appropriate.

Note that the present technique can also take the following configurations.
(1) A simulation system comprising
   a first information processing apparatus, and a second information processing apparatus that provide simulation of surgery in cooperation with each other, wherein
   the first information processing apparatus and the second information processing apparatus transmit and receive simulation data to and from each other via an all-optical communication network.
(2) The simulation system according to (1), wherein
   the first information processing apparatus and the second information processing apparatus transmit and receive the simulation data to and from each other with remote DMA (Direct Memory Access).
(3) The simulation system according to (1) or (2), wherein
   the second information processing apparatus calculates a simulation model including a soft tissue.
(4) The simulation system according to (3), wherein
   the calculation of the simulation model includes calculation of deformation of the soft tissue.
(5) The simulation system according to (4), wherein the soft tissue includes an organ.
(6) The simulation system according to (4) or (5), wherein
   the second information processing apparatus calculates a contact force generated by interaction between the soft tissue and a surgical instrument.
(7) The simulation system according to (6), wherein
   the first information processing apparatus controls a haptic device operated by a user such that the contact force calculated by the second information processing apparatus is fed back to the user.
(8) The simulation system according to (7), wherein
   the second information processing apparatus calculates the contact force at a predetermined period,
   the first information processing apparatus controls the haptic device such that the contact force calculated by the second information processing apparatus is fed back to the user in the period, and
   the predetermined period is shorter than a calculation period in a case in which it is assumed that the first information processing apparatus has performed the calculation of the simulation model including the calculation of the contact force and is same as or longer than a control period of the haptic device.
(9) The simulation system according to (8), wherein
   the predetermined period is a period equal to or higher than 60 Hz and approximately same as 1 kHz.
(10) The simulation system according to (8) or (9), wherein
   the haptic device includes a joint in which at least one of acceleration and force is controlled.
(11) The simulation system according to any one of (8) to (10), wherein
   the haptic device includes a joint in which at least one of a six-axis force sensor and a torque sensor is provided.
(12) The simulation system according to any one of (1) to (11), wherein
   the first information processing apparatus is a plurality of first information processing apparatuses used by different users.
(13) The simulation system according to any one of (1) to (6), wherein
   the surgery is surgery of controlling, with a master-slave scheme, a robot arm supporting a surgical instrument, and
   a result of the simulation is reflected in control of the robot arm.
(14) An information processing apparatus that provides simulation of surgery in cooperation with another information processing apparatus, the information processing apparatus comprising:
   a processing unit that calculates a simulation model including a soft tissue; and
   an optical transmission control unit that controls transmission and reception of simulation data to and from the other information processing apparatus via an all-optical communication network.
(15) An information processing method for providing simulation of surgery by a first information processing apparatus and a second information processing apparatus cooperating with each other, the information processing method comprising,
   by the first information processing apparatus and the second information processing apparatus, transmitting and receiving simulation data to and from each other via an all-optical communication network.

### Reference Signs List

1 INFORMATION PROCESSING APPARATUS
11 PROCESSING UNIT
12 STORAGE UNIT
13 IF UNIT
14 COMMUNICATION CONTROL UNIT
15 OPTICAL TRANSMISSION CONTROL UNIT
16 OPTICAL TRANSMISSION DEVICE
2 INFORMATION PROCESSING APPARATUS
21 PROCESSING UNIT
22 STORAGE UNIT
24 COMMUNICATION CONTROL UNIT
25 OPTICAL TRANSMISSION CONTROL UNIT
26 OPTICAL TRANSMISSION DEVICE
27 VIRTUAL LAYER
3 LEADER APPARATUS (MASTER APPARATUS)
4 FOLLOWER APPARATUS (SLAVE APPARATUS)
41 PROCESSING UNIT
43 IF UNIT
44 COMMUNICATION CONTROL UNIT
6 HAPTIC DEVICE
61 JOINT
611 MOTOR
612 ENCODER
613 SENSOR
614 MOTOR CONTROL UNIT
615 DRIVER
62 HAPTICS CONTROL UNIT
7 MONITOR
8 CAMERA
9 ROBOT ARM
100 SIMULATION SYSTEM
1000 COMPUTER
1050 BUS
1100 CPU/GPU
1200 RAM
1300 ROM
1400 HDD
1450 PROGRAM DATA
1500 COMMUNICATION INTERFACE
1600 INPUT/OUTPUT INTERFACE
1650 INPUT/OUTPUT DEVICE
C CAMERA
F FIELD OF VIEW
N ALL-OPTICAL COMMUNICATION NETWORK
O SOFT TISSUE
R ROBOT ARM
T SURGICAL INSTRUMENT
U USER

## Claims

1. A simulation system comprising
a first information processing apparatus, and a second information processing apparatus that provide simulation of surgery in cooperation with each other, wherein
the first information processing apparatus and the second information processing apparatus transmit and receive simulation data to and from each other via an all-optical communication network.

2. The simulation system according to claim 1, wherein
the first information processing apparatus and the second information processing apparatus transmit and receive the simulation data to and from each other with remote DMA (Direct Memory Access).

3. The simulation system according to claim 1, wherein
the second information processing apparatus calculates a simulation model including a soft tissue.

4. The simulation system according to claim 3, wherein
the calculation of the simulation model includes calculation of deformation of the soft tissue.

5. The simulation system according to claim 4, wherein
the soft tissue includes an organ.

6. The simulation system according to claim 4, wherein
the second information processing apparatus calculates a contact force generated by interaction between the soft tissue and a surgical instrument.

7. The simulation system according to claim 6, wherein
the first information processing apparatus controls a haptic device operated by a user such that the contact force calculated by the second information processing apparatus is fed back to the user.

8. The simulation system according to claim 7, wherein
the second information processing apparatus calculates the contact force at a predetermined period,
the first information processing apparatus controls the haptic device such that the contact force calculated by the second information processing apparatus is fed back to the user in the period, and
the predetermined period is shorter than a calculation period in a case in which it is assumed that the first information processing apparatus has performed the calculation of the simulation model including the calculation of the contact force and is same as or longer than a control period of the haptic device.

9. The simulation system according to claim 8, wherein
the predetermined period is a period equal to or higher than 60 Hz and approximately same as 1 kHz.

10. The simulation system according to claim 7, wherein
the haptic device includes a joint in which at least one of acceleration and force is controlled.

11. The simulation system according to claim 7, wherein
the haptic device includes a joint in which at least one of a six-axis force sensor and a torque sensor is provided.

12. The simulation system according to claim 1, wherein
the first information processing apparatus is a plurality of first information processing apparatuses used by different users.

13. The simulation system according to claim 1, wherein
the surgery is surgery of controlling, with a master-slave scheme, a robot arm supporting a surgical instrument, and
a result of the simulation is reflected in control of the robot arm.

14. An information processing apparatus that provides simulation of surgery in cooperation with another information processing apparatus, the information processing apparatus comprising:
a processing unit that calculates a simulation model including a soft tissue; and
an optical transmission control unit that controls transmission and reception of simulation data to and from the other information processing apparatus via an all-optical communication network.

15. An information processing method for providing simulation of surgery by a first information processing apparatus and a second information processing apparatus cooperating with each other, the information processing method comprising,
by the first information processing apparatus and the second information processing apparatus, transmitting and receiving simulation data to and from each other via an all-optical communication network.
